# EUROPÄISCHE PATENTSCHRIFT

(11) **EP 3 011 281 B1**
(45) Veröffentlichungstag und Bekanntmachung des Hinweises auf die Patenterteilung: **04.11.2020**
(21) Anmeldenummer: 14726955.9
(22) Anmeldetag: 26.05.2014
(51) Int. Cl.: G01G 9/00, B65B 3/28, G01N 23/12, G01N 33/15, G01G 17/00, A61J 3/07

(54) **VORRICHTUNG UND VERFAHREN ZUR GEWICHTSBESTIMMUNG INSBESONDERE EINES MIT PRODUKT BEFÜLLTEN BEHÄLTNISSES**
DEVICE AND METHOD FOR DETERMINING WEIGHT, IN PARTICULAR THE WEIGHT OF A CONTAINER FILLED WITH PRODUCT
DISPOSITIF ET PROCÉDÉ PERMETTANT DE DÉTERMINER LE POIDS EN PARTICULIER D'UN CONTENANT REMPLI DE PRODUIT

(30) Priorität: 19.06.2013 DE 102013211526
(43) Veröffentlichungstag der Anmeldung: 27.04.2016
(73) Patentinhaber: Syntegon Technology GmbH, 71332 Waiblingen (DE)
(72) Erfinder: MAGA, Iulian, 71642 Ludwigsburg (DE); VOGT, Martin, 73614 Schorndorf (DE); SCHLIPF, Jens, 71691 Freiberg A. N. (DE)
(74) Vertreter: DREISS Patentanwälte PartG mbB
(86) Internationale Anmeldenummer: PCT/EP2014/060801
(87) Internationale Veröffentlichungsnummer: WO 2014/202340

(56) Entgegenhaltungen:
- WO-A1-2006/106012
- WO-A1-2012/013368
- JP-A- H10 239 251
- JP-A- H10 239 251
- JP-A- 2006 308 467
- JP-A- 2006 308 467

## Beschreibung

Die Erfindung geht aus von einer Vorrichtung und einem Verfahren zur Gewichtsbestimmung von pharmazeutischen Produkten mittels einer Röntgenstrahlungsquelle nach der Gattung der unabhängigen Ansprüche. Eine gattungsgemäße Vorrichtung und Verfahren sind bereits aus der WO 2012/013368 A1 bekannt. Eine Röntgenstrahlungsquelle erzeugt einen Strahlungskegel, der wenigstens ein pharmazeutisches Produkt durchstrahlt. Ein Sensorelement erfasst die Strahlung des durchstrahlten pharmazeutischen Produkts und führt es einer Auswerteeinrichtung zu. Im Strahlungsgang des Strahlungskegels ist ein Referenzobjekt angeordnet, wobei die Strahlung des durchleuchteten Referenzobjekts mittels Sensorelements erfasst und der Auswerteeinheit zugeführt wird, wobei das pharmazeutische Produkt und das Referenzobjekt im Bezug auf den Strahlungskegel in nicht überdeckender Anordnung zueinander im Strahlungskegel positioniert sind.

Es ist Aufgabe der vorliegenden Erfindung, die Genauigkeit einer Gewichtsermittlung weiter zu verbessern. Diese Aufgabe wird gelöst durch die Merkmale der unabhängigen Ansprüche.

Aus der JP 2006308467A ist eine Röntgeninspektionseinrichtung bekannt, die bestimmte physikalische Größen ermittelt, die relevant sind für die Größe oder Masse bzw. Gewicht des inspizierten Gegenstands. Wenn das zu messende Objekt sich in einem Behälter befindet und der Behälter nicht in die Masse des zu messenden Objekts eingehen soll, ist der entsprechende Einfluss des Behälters herauszurechnen.

Aus der JP H10 239251 A ist eine Vorrichtung zur Auswertung einer Ablagerung innerhalb einer Rohrleitung bekannt. Eine Röntgenstrahlungsquelle durchstrahlt das Rohr. Ein auf der gegenüberliegenden Seite angeordneter Sensor nimmt ein entsprechendes Bild auf zur weiteren Analyse des Zustandes der Ablagerung innerhalb des Rohrs. Die Auswertung hängt von der speziellen Geometrie des Sensors ab, da lediglich ein Teil der Rohrleitung durchleuchtet wird.

### Vorteile der Erfindung.

Die erfindungsgemäße Vorrichtung und das erfindungsgemäße Verfahren gemäß den Merkmalen der unabhängigen Ansprüche haben demgegenüber den Vorteil, dass die Messgenauigkeit bezogen auf das Nettogewicht deutlich erhöht werden kann. Damit ist eine 100%ige Inline-Nettogewichtsbestimmung während des Normalbetriebs mit hoher Genauigkeit ohne den Einsatz einer gravimetrischen Wägezelle möglich. Denn die erfindungsgemäße Vorrichtung und das erfindungsgemäße Verfahren nutzt spezielle Informationen aus insbesondere digitalen Bildern der mit pharmazeutischen Produkten befüllten Behältnisse, um das Nettogewicht mit einer höheren Genauigkeit zu bestimmen als dies mit einer üblichen Bruttogewichtsbestimmung möglich wäre. Dies wird insbesondere dadurch möglich, dass auf Basis des frei durchleuchtbaren Teils eines Behältnisses, insbesondere das Teil eines Behältnisses, in dem sich kein Produkt befindet, ermittelt wird, wie schwer ein leeres Behältnis ist. Anschließend wird dieser ermittelte Taragewichtswert vom Bruttogewicht abgezogen. Das Ergebnis ist das Nettogewicht des in dem Behältnis befindlichen Produkts. Die Auswertung kann mit Hilfe nur eines einzigen Röntgenbilds erfolgen. Damit wird gezielt das in erster Linie interessierende Nettogewicht des Produkts ermittelt und nicht mehr lediglich das Bruttogewicht von Behältnis und Produkt wie im Stand der Technik. Dies ist insbesondere bei kleinen Füllmengen von besonderem Vorteil, da in diesen Fällen das Gewicht des Behältnisses einen großen Einfluss auf die Genauigkeit der Gewichtsermittlung besitzt. So können bei kleinen Füllmengen Fertigungsschwankungen des Taragewichts leerer Behältnisse größer sein als maximal zulässige Füllmengenschwankungen. Dies kann dazu führen, dass Behältnisse mit korrekter Füllmenge des Produkts als nicht korrekt befüllt aussortiert werden, weil das Taragewicht des Behältnisses die Gewichtstoleranz ausgeschöpft hat. Umgekehrt kann dies auch dazu führen, dass ein mit zu viel oder zu wenig befülltem Produkt befülltes Behältnis fälschlicher Weise als in Ordnung erkannt wird, weil sich die tolerierte Behältnisgewichtsschwankung gegenläufig verhält und die Fehldosierung ausgleicht. Diesen nicht akzeptablen Fällen wirkt die beschriebene Nettogewichtsermittlung besonders vorteilhaft entgegen.

In einer zweckmäßigen Weiterbildung ist vorgesehen, dass die Auswerteeinrichtung das Taragewicht des Behältnisses ermittelt, indem die Auswerteeinrichtung ein Maß für eine Fläche der Auswerteregion in das Verhältnis setzt zu einem Maß für eine Fläche einer Kontur des gesamten durchstrahlten Behältnisses. Besonders bevorzugt werden als Maß für eine Fläche der Auswerteregion bzw. der Kontur solche Bildpunkte des Sensors verwendet, die innerhalb der Auswerteregion bzw. innerhalb der Kontur liegen. In besonders einfacher Weise lässt sich die Information des flächigen Sensors zur Extrapolation des Taragewichts auf das gesamte Behältnis nutzen, ohne dass zusätzliche Sensoren oder Messungen notwendig wären. Dadurch vereinfacht sich der Aufbau der Vorrichtung.

In einer zweckmäßigen Weiterbildung ist vorgesehen, dass die Auswerteeinrichtung innerhalb der Auswerteregion und/oder der Kontur liegende Grauwerte und/oder Bildpunkte des Sensors auswertet zur Ermittlung des Gewichts. Durch eine gezielte Auswertung der interessierenden Bereiche kann die Genauigkeit der Gewichtsermittlung weiter erhöht werden. Die Nettogewichtsermittlung verfälschende Größen lassen sich leicht heraus rechnen.

In einer zweckmäßigen Weiterbildung ist vorgesehen, dass ein Einlernen der Vorrichtung auf die verwendeten Behältnisse, insbesondere leere Behältnisse, erfolgt. Hierzu wird bevorzugt eine Referenzlinie des Behältnismaterials erstellt, wobei der Bezug zum gravimetrischen Gewicht mit Hilfe einer sehr genauen Waage erfolgen könnte (Tarajustierung).

In einer zweckmäßigen Weiterbildung ist vorgesehen, dass die Vorrichtung auf Behältnisse mit befülltem Produkt eingelernt werden. Hierbei wird die Referenzlinie eines Behältnisses mit Produkt erstellt, wobei der Bezug zum gravimetrischen Gewicht mit Hilfe einer sehr genauen Waage hergestellt werden kann (Bruttojustierung).

In einer zweckmäßigen Weiterbildung ist vorgesehen, dass die Vorrichtung Mittel zur Bilderzeugung des zu prüfenden Behältnisses umfasst. Es wird gezielt eine bestimmte Auswerteregion vorgegeben. Diese ist bevorzugt in einem solchen Behältnisbereich, der keine Befüllung mit einem Produkt aufweist. Anschließend wird diese Region des frei durchleuchtbaren Teils des Behältnisses ausgewertet. Dies erfolgt anhand dieses frei durchleutbaren Teils des Behältnisses sowie der ermittelten Referenzlinie im Rahmen der Tarajustierung und eine Hochrechnung auf das Leergewicht eines Behältnisses (Taragewicht).

In einer zweckmäßigen Weiterbildung ist vorgesehen, dass unter Rückgriff auf das bereits ermittelte Bild nun eine Auswertung des gesamten Behältnisses erfolgen kann inklusive des Produkts. Basierend auf dieser Information und der Referenzlinie wie im Rahmen der Bruttojustierung ermittelt wird das Bruttogewicht bestimmt.

In einer zweckmäßigen Weiterbildung ist vorgesehen, dass das Nettogewicht des zu prüfenden Behältnisses ermittelt wird, indem die Differenz gebildet wird aus dem Bruttogewicht und dem Taragewicht wie in den vorangegangen Verfahrensschritten ermittelt.

In einer zweckmäßigen Weiterbildung ist vorgesehen, dass zumindest ein Referenzobjekt vorgesehen ist, das im Strahlungsgang der Röntgenstrahlungsquelle angeordnet ist, und das unterschiedliche Dicken aufweist, wobei zur Nettogewichtsermittlung die Auswerteeinheit den Grauwerten des Differenzbilds jeweils eine dem Grauwert des Referenzobjekts entsprechende Dicke des Referenzobjekts zuordnet. Gerade durch die Verwendung eines Referenzobjekts mit unterschiedlichen Dicken, das im laufenden Betrieb ebenfalls zusammen mit dem Behältnis durchstrahlt wird, lassen sich die Grauwerte verlässlich in ein Nettogewicht überführen. Dies kann mit Hilfe der entsprechenden Dicken, Überführung in ein Volumen und Zuordnung zu einem Nettogewicht mittels der Referenzlinie in besonders einfacher und genauer Weise erfolgen.

In einer zweckmäßigen Weiterbildung ist vorgesehen, dass zumindest ein zweites Referenzobjekt vorgesehen ist, das im Strahlungsgang der Röntgenstrahlungsquelle angeordnet ist, und das unterschiedliche Dicken aufweist. Dabei könnte eines der Referenzobjekte auf das Material eines Leerbehältnisses und das andere auf die Behältnisfüllung angepasst sein. Je mehr sich Leerbehältnis und Produkt bezüglich ihrer Absorptionseigenschaften voneinander unterscheiden, desto größere Genauigkeitssteigerungen können damit erzielt werden. Bei der Tarajustierung würde das auf das Leerbehältnis abgestimmte Referenzobjekt verwendet zur Erstellung der Referenzlinie. Bei der Bruttojustierung würde das auf die Behältnisfüllung abgestimmte weitere Referenzobjekt verwendet zur Erstellung der Referenzlinie. Bei der sich anschließenden eigentlichen Messung wird die Leerbehältnisextrapolation wieder mit Hilfe des auf das Leerbehältnis abgestimmten Referenzobjekts , die Bruttomessung mithilfe des auf die Behältnisfüllung abgestimmten weiteren Referenzobjekts durchgeführt. Die Umrechnung in virtuelles Volumen und damit in das Gewicht wird somit differenziert, je nach dem betrachteten Material, durchgeführt und führt zu einer nochmals verbesserten Genauigkeit.

In einer zweckmäßigen Weiterbildung ist ein Aufnahmemittel, insbesondere ein Kapselhalter, vorgesehen zur Aufnahme zumindest eines Behältnisses, wobei das Behältnis über ein Fördermittel zu einer Bearbeitungsstation und/oder der Röntgenstrahlungsquelle zugeführt ist zur Erzeugung des Bildes. Diese Anordnung eignet sich insbesondere für Behältnisse, die mit hoher Geschwindigkeit unterschiedlichen Arbeitsstationen zugeführt werden. Dadurch lassen sich hohe Ausbringungsgeschwindigkeiten erreichen.

Weitere zweckmäßige Weiterbildungen ergeben sich aus weiteren abhängigen Ansprüchen und aus der Beschreibung.

### Zeichnung

Ein Ausführungsbeispiel der erfindungsgemäßen Vorrichtung und des erfindungsgemäßen Verfahrens sind in der Zeichnung dargestellt und werden nachfolgend näher beschrieben.

Es zeigen:
Fig. 1 das Grundprinzip einer Bilderzeugung, welche auf Röntgenstrahlung basiert,
Fig. 2 ein teilweise befülltes Behältnis,
Fig. 3 ein Bild eines befüllten Behältnisses mit festgelegten Auswerteregionen,
Fig. 4 eine erfindungsgemäße Vorrichtung,
Fig. 5 ein Flussdiagramm des erfindungsgemäßen Verfahrens,
Fig. 6 ein anderes befülltes Behältnis,
Fig. 7 eine nochmals modifizierte Vorrichtung zur Gewichtsbestimmung,
Fig. 8 ein Bild mehrerer durchstrahlter Behältnisse und des Referenzobjekts bei einer Anordnung nach Figur 7.

Mehrere Behältnisse 3 befinden sich in einem Stahlungsgang 36 einer Röntgenstrahlungsquelle 28, die die Behältnisse 3 durchstrahlt. Dahinter befindet sich ein Sensor 30 mit einer Sensoroberfläche 32, die der Röntgenstrahlungsquelle 28 zugewandt ist. Der Sensor 30 dient der Erstellung eines Röntgenbildes bzw. Bildes 12 des durchstrahlten Behältnisses. Die Sensoroberfläche 32 umfasst bevorzugt eine Vielzahl von strahlungsempfindlichen Sensorelementen wie beispielsweise CCD- oder CMOS Sensoren, die abhängig von der auftreffenden Stahlung jeweils ein Ausgangssignal abgeben, von dem nachfolgend als Grauwert gesprochen wird. Hierzu ist beispielsweise über den Pixeln des CMOS-Sensors eine Szintillationsschicht aufgebracht, die die Röntgenenergie in sichtbares Licht umwandelt. Das Pixel des Sensors erzeugt abhängig von der Lichtmenge eine Ladung (analog), die bevorzugt in ein digitales Signal ("Grauwert") umgewandelt wird, das für die Gewichtsermittlung herangezogen wird. Über die Ausgangssignale der flächig angeordneten Sensorelemente wird das Bild 12 erzeugt. Prinzipiell können auch andere Strahlungserfassungsmittel als Sensoren verwendet werden.

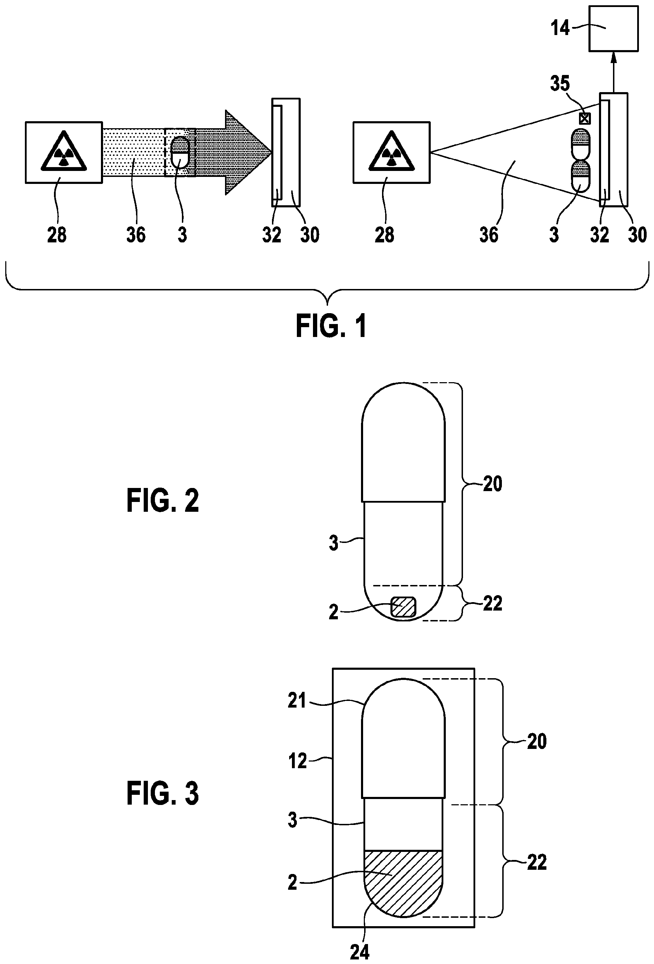

Bei dem rechten Ausführungsbeispiel der Figur 1 befindet sich auch ein Referenzelement 35 im Strahlungsgang 36 der Röntgenstrahlungsquelle 28. Das Referenzelement 35 befindet sich in nicht überdeckender Anordnung neben den Behältnissen 3 und wird ebenfalls durchstrahlt. Das hierbei entstehende Bild 12 nimmt der Sensor 30 ebenfalls auf.

Bei dem Behältnis 3 gemäß Figur 2 handelt es sich beispielhaft um eine Kapsel, in der sich zum Teil ein Produkt 2 wie beispielsweise ein Pharmazeutikum befindet. Somit weist das Behältnis 3 einen frei durchleuchtbaren Bereich 20, in dem sich kein Produkt 2 befindet, und einen nicht frei durchleuchtbaren Bereich 22, in welchem sich das Produkt 2 befindet, auf.

Bei der Figur 3 weist das Behältnis 3 wiederum einen nicht frei durchleuchtbaren Bereich 22 sowie einen frei durchleuchtbaren Bereich 20 auf. Es wird nun eine Auswerteregion 21 für den frei durchleuchtbaren Bereich 20 festgelegt in der Weise, dass sich innerhalb der Auswerteregion 21 sicher kein Produkt 2 befindet.

Außerdem wird eine Kontur 24 des Behältnisses 3, die der Außenkontur entspricht, über eine Schwellwertauswertung der Graustufen festgelegt.

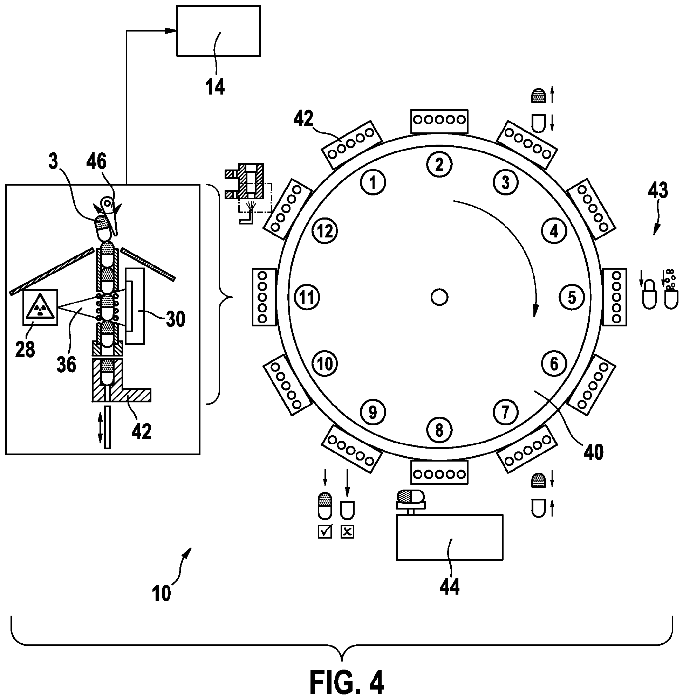

In Figur 4 ist die Vorrichtung 10 gezeigt, die sich für die Durchführung des beschriebenen Verfahrens eignet. Dies ist exemplarisch am konkreten Beispiel einer Kapselfüllmaschine beschrieben, für die sich die Erfindung besonders eignet, hierauf jedoch nicht eingeschränkt ist.

Eine Maschine zum Füllen und Verschließen von aus einem Kapselunterteil und einer aufgesteckten Kappe bestehenden Behältnis 3 hat ein zwölfteiliges, schrittweise um eine vertikale Achse gedrehtes Förderrad 40, an dessen Stationen 1 bis 12 an der Umlaufstrecke die einzelnen Behandlungseinrichtungen angeordnet sind. Auch andere Teilungen des Förderrades 40 wie beispielsweise eine 15-teilige wären denkbar. Bei den Stationen 1 und 2 werden die zu füllenden, leeren Behältnisse 3 ungeordnet aufgegeben sowie ausgerichtet. Die Behältnisse 3 werden anschließend geordnet einem Kapselhalter 42 eines Förderrad 40 zugeführt. In Station 3 werden die noch verschlossenen Behältnisse 3 geöffnet. In Station 5 erfolgt die Befüllung des Behältnisses 3 mit dem Produkt 2. Bei Station 7 wird das befüllte Behältnis 3 verschlossen. Bei Station 8 werden die Behältnisse 3 durch eine Wiegeeinrichtung 44 gewogen. Die Wiegeeinrichtung 44 kann unterschiedliche Messungen vornehmen. Zum einen kann die Wiegeeinrichtung 44 zur Initialisierung eine Verwiegung des leeren bzw. gefüllten Behältnisses 3 vornehmen. Die Wiegeeinrichtung 44 könnte auch zur Vergleichswiegung während der laufenden Produktion zur Überprüfung des beschriebenen, auf Röntgenstrahlung basierenden Messystems herangezogen werden. In Station 9 erfolgt eine Schließdruckkontrolle des verschlossenen Behältnisses 3. Abhängig von der Prüfung in Station 9 werden fehlerhafte Behältnisse 3 in Station 10, ordnungsgemäße erst in Station 11 ausgestoßen. In Station 11 gelangen die Behältnisse 3 von dem Kapselhalter 42 nach oben in den Strahlungsgang 36 einer Röntgenstrahlungsquelle 28. Abhängig von dem in Schritt 103 ermittelten Gewicht des Produkts 3 können mit Hilfe einer Weiche 46 fehlerhafte Behältnisse 3 noch ausgesondert werden.

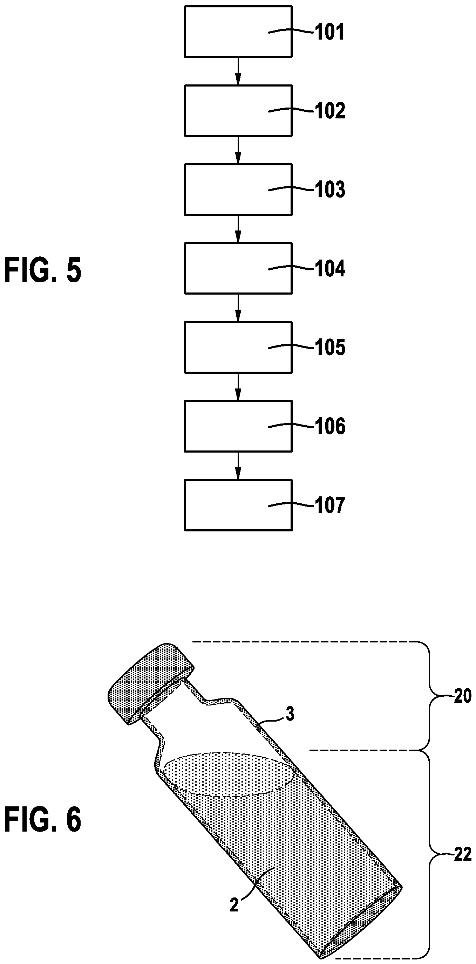

Bei dem Ausführungsbeispiel gemäß Figur 6 ist als Behältnis 3 eine Ampulle vorgesehen, welche mit einem flüssigen Produkt 2 gefüllt ist. Auch für dieses Produkt lassen sich ein nicht frei durchleuchtbarer Bereich 22 und ein frei durchleuchtbarer Bereich 20 definieren.

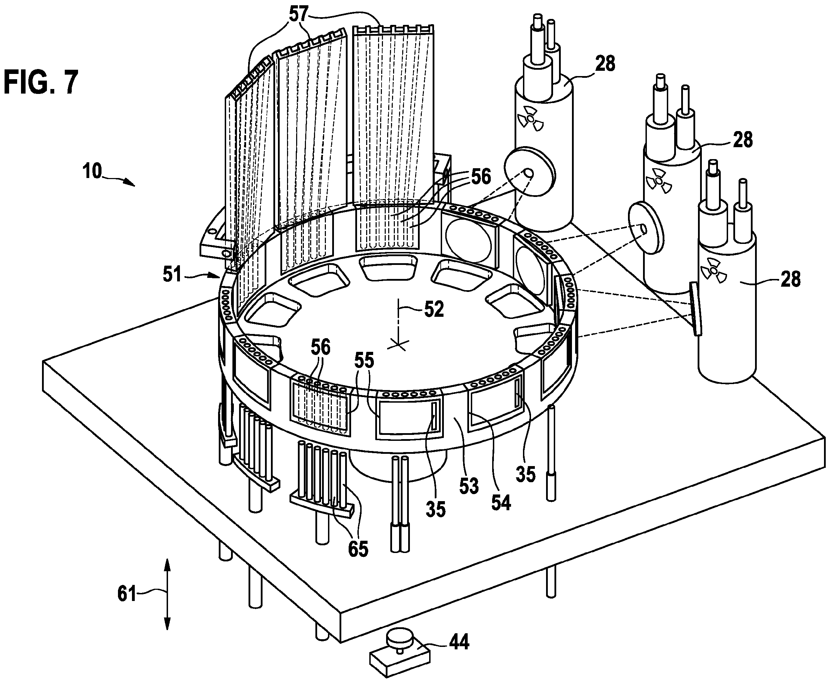

In der Fig. 7 ist eine nochmals modifizierte Vorrichtung 10 zur Gewichtsbestimmung dargestellt. Die Vorrichtung 10 weist ein Förderrad 51 auf, das in einer vertikal angeordneten Drehachse 52 schrittweise gedreht wird. An einer ringförmigen, vertikal ausgerichteten Außenwand 53 des Förderrads 51 sind in gleichmäßigen Winkelabständen Aufnahmen 54 zur austauschbaren Befestigung von Formatteilen 55 ausgebildet. In den Formatteilen 55 sind jeweils mehrere, insbesondere ebenfalls vertikal ausgerichtete, als Aufnahmen für die Behältnisse 3 wirkende Aufnahmebohrungen 56 ausgebildet, die jeweils eine Höhe bzw. Länge aufweisen, die es ermöglicht, jeweils mehrere Behältnisse 3 übereinander als Reihe stehend in den Aufnahmebohrungen 56 aufzunehmen. Außerdem weisen die Formatteile 55 jeweils zumindest ein Referenzobjekt 35 auf, das neben den Aufnahmebohrungen 56 ebenfalls im Strahlungsgang 36 der Röntgenstrahlungsquelle 28 angeordnet ist. Die Formatteile 55 bestehen aus einem Material, insbesondere aus Kunststoff, das für die Röntgenstrahlung durchlässig ist. Die Aufnahmebohrungen 56 der Formatteile 55 werden mittels schachtförmiger Zuführrinnen 57 aus einem nicht dargestellten Massenspeicher mit den Behältnissen 3 befüllt, wobei im Bereich der Zuführrinnen 57 entsprechend der Vorrichtung 10 jeweils Sperreinrichtungen angeordnet sind. Im dargestellten Ausführungsbeispiel sind am Förderweg des Förderrads 51 außerhalb dessen Außenumfangs mehrere Röntgenstrahlungsquellen 28 angeordnet. Hierbei entspricht die Anzahl der Röntgenstrahlungsquellen 28 bevorzugt der Anzahl der Zuführrinnen 57, so dass das Förderrad 51 beispielsweise beim Vorhandensein von drei Zuführrinnen 57 jeweils schrittweise um einen Drehwinkelbereich weitergedreht wird, der der Teilung von drei Zuführrinnen 57 entspricht. Um einzelne Behältnisse 3, die als "schlecht" erkannt wurden, aus dem Förderrad 51 bzw. den Aufnahmebohrungen 56 der Formatteile 55 ausscheiden zu können, sind am weiteren Förderweg des Förderrads 51 nach den Röntgenstrahlungsquellen 28 entsprechend des Doppelpfeils 61 auf- und abbewegbare Ausscheidestößel 65 vorgesehen, die in die als Stufenbohrungen ausgebildeten Aufnahmebohrungen 56 der Formatteile 55 einfahren können, um damit die im Bereich der Aufnahmebohrung 56 befindlichen Behältnissen 3 auszuscheiden. Die Vorrichtung 10 umfasst auch eine Wiegeeinrichtung 44 zum nachfolgend beschriebenen Einlernen.

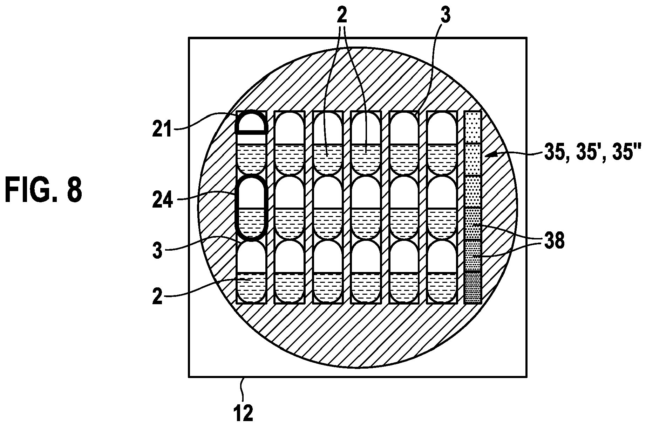

Das bei der Anordnung nach Figur 7 entstehende Bild 12 gemäß Figur 8 zeigt zum einen das Röntgenbild mehrerer neben- und übereinandergeordneter Behältnisse 3. Auf der rechten Seite ist das Bild des durchstrahlten Referenzobjekts 35 sichtbar, das unterschiedliche Graustufen 38 besitzt. Das Referenzobjekt 35 ist treppenstufenförmig mit unterschiedlichen Dicken aufgebaut. Bei den jeweiligen unterschiedlichen Dicken ergeben sich unterschiedliche Graustufen 38 wie nachfolgend noch näher ausgeführt. Beispielhaft ist bereits die Auswerteregion 21 und die Kontur 24 des Behältnisses 3 festgelegt bzw. ermittelt.

In einer alternativen Ausgestaltung ist vorgesehen, dass zumindest ein zweites Referenzobjekt 35' vorgesehen ist, das im Strahlungsgang 36 der Röntgenstrahlungsquelle 28 angeordnet ist, und das unterschiedliche Dicken aufweist. Dabei könnte eines der Referenzobjekte 35', 35" auf das Material eines Leerbehältnisses 3 und das andere auf die Behältnisfüllung 2 angepasst sein. Je mehr sich Leerbehältnis 3 und Produkt 2 bezüglich ihrer Absorptionseigenschaften voneinander unterscheiden, desto größere Genauigkeitssteigerungen können damit erzielt werden. Bei der Tarajustierung würde das auf das Leerbehältnis 3 abgestimmte Referenzobjekt 35' verwendet zur Erstellung der Referenzlinie. Bei der Bruttojustierung würde das auf die Behältnisfüllung abgestimmte weitere Referenzobjekt 35" verwendet zur Erstellung der Referenzlinie. Bei der sich anschließenden eigentlichen Messung wird die Leerbehältnisextrapolation wieder mit Hilfe des auf das Leerbehältnis abgestimmten Referenzobjekts 35', die Bruttomessung mithilfe des auf die Behältnisfüllung abgestimmten weiteren Referenzobjekts 35" durchgeführt. Die Umrechnung in virtuelles Volumen und damit in das Gewicht wird somit differenziert, je nach dem betrachteten Material, durchgeführt und führt zu einer nochmals verbesserten Genauigkeit.

Das Bild 12 wird zum einen hinsichtlich der Kontur 24 des Behältnisses 3 ausgewertet. Hierzu werden die den jeweiligen Bildpunkten zugeordneten Grauwerte ausgewertet. Diese Grauwerte werden mit solchen Grauwerten verglichen, die bei Durchstrahlung des bekannten Referenzobjekts 35 entstehen.

Prinzipiell erfolgt eine Gewichtsermittlung gemäß der in Figur 1 gezeigten Anordnung wie nachfolgend allgemein beschrieben. Vorteilhafterweise besteht das Referenzobjekt 35 aus einem Material, das ähnliche atomare Eigenschaften aufweist wie das zu durchstrahlende pharmazeutische Produkt 2, d.h. insbesondere für die Röntgenstrahlung dieselben Dämpfungseigenschaften aufweist. Das Referenzobjekt 35 weist über den Querschnitt betrachtet unterschiedliche Dicken auf. Das Referenzobjekt 35 kann keilförmig ausgebildet sein. Alternativ weist das Referenzobjekt 35eine Reihe von Stufen auf, die eine diskrete Änderung der Dicke des Referenzobjekts 35 bewirken. Vorzugsweise ist es vorgesehen, dass die Dämpfung (Grauwert) des Referenzobjekts 35 an einer Stelle größer und an einer anderen Stelle kleiner ist als die Dämpfung durch das pharmazeutische Produkt 2. Durch die angesprochene geometrische Gestaltung des Referenzobjekts 35 weisen diese unterschiedliche Dicken auf, die bei einer Durchstrahlung mittels der Röntgenstrahlungsquelle 28 am Sensorelement 30 unterschiedliche Graustufen 38 erzeugen. Einzelne Stufen des Referenzobjekts 35 weisen unterschiedliche Grauwerte 38 auf.

Die Gewichtsbestimmung des in einem Behältnis 3 befindlichen Produkt 2 erfolgt prinzipiell wie folgt: In einem nicht dargestellten, vorab stattfindenden Justierprozess wird ein Bild des Referenzobjekts 35 aufgenommen, und dessen von der Sensoreinrichtung 30 erfasste Grauwerte 38 werden aufgrund der bekannten geometrischen Ausbildung des Referenzobjekts 35 den Dicken des Referenzobjekts 35 zugeordnet. Mit anderen Worten gesagt bedeutet dies, dass aufgrund eines bestimmten Grauwertes 38 des Referenzobjekts 35 auf eine bestimmte Dicke des Referenzobjekts 35 an einer bestimmten Stelle geschlossen wird. Ferner kann aufgrund der bekannten Geometrie des Referenzobjekts 35 damit einem bestimmten Grauwert 38 des Referenzobjekts 35 eine bestimmte Dickte zugeordnet werden. Diese vorab in dem Kalibrierprozess ermittelten Grauwerte 38 sowie deren geometrische Zuordnung an dem Referenzobjekt 35 sind in der Auswerteeinrichtung 14 abgespeichert. Soll nun beispielsweise das Gewicht erfasst bzw. überprüft werden, so wird das von dem Sensorelement 30 erfasste Bild 12 des Behältnisses 3 in einzelne Bildpunkte (Pixel) aufgeteilt. Dieser Bildpunkt stellt eine bestimmte Fläche, zum Beispiel ein Quadrat mit einer Kantenlänge von 100µm, dar. Dann wird für jeden Bildpunkt des Behältnisses 3 der erfasste Grauwert des Bildpunktes einem (identischen) Grauwert 38 am Referenzobjekt 35 zugeordnet. Diesem Grauwert kann (aufgrund der Zuordnung der Dicken zu den Grauwerten 38 am Referenzobjekt 35) eine bestimmte Dicke zugeordnet werden. Nachdem dies Bildpunkt für Bildpunkt geschehen ist, wird aus einzelnen Dicken eine mittlere Dicke ermittelt. Diese mittlere Dicke wird nun mit der Gesamtanzahl der Bildpunkte und deren bekannte Fläche multipliziert, so dass ein virtuelles Volumen des Produkts 2 bestimmt werden kann. Aus dem virtuellen Volumen kann zuletzt das Gewicht des in dem Behältnis 3 befindlichen Produkts 2 ermittelt werden.

Während des Durchleuchtens der insbesondere pharmazeutischen Produkte 2 wird gleichzeitig auch ein Bild des betreffenden Referenzobjekts 35 mit aufgenommen. Dadurch lassen sich Änderungen der Graustufen am Referenzobjekt 35 an zeitlich aufeinanderfolgenden Bildern ermitteln, die aufgrund von Störungen des Systems oder aufgrund externer Störeinflüsse auftreten können. Sollte beispielsweise festgestellt werden, dass sich der Grauwert 38 des Referenzobjekts 35 an einer bestimmten Stufe ändert, so kann die Auswerteeinrichtung 14 diesen erfassten aktuellen Grauwert mit einem Korrekturfaktor oder Offset belegen, was den aktuellen Grauwert dem ursprünglichen Grauwert anpasst und somit die Störeinflüsse egalisiert. Basierend auf dieser generellen Vorgehensweise erfolgt nachfolgend beschriebene erfindungsgemäße Nettogewichtsermittlung mittels Leerbehältnisextrapolation.

Die erfindungsgemäße Vorrichtung 10 arbeitet wie folgt. In einem ersten Schritt 101 wird das System auf die verwendeten leeren Behältnisse 3 eingelernt. Hierzu werden zumindest ein leeres Behältnis 3 und das daneben angeordnete Referenzelement 35 durchstrahlt. Der dahinter liegende Sensor 30 erfasst die auf die Sensoroberfläche 32 fallende Strahlung in Form eines Bildes 12 mit unterschiedlichen Grauwerten. Dieses Bild 12 wird zuerst zur Konturerfassung ausgewertet, indem z.B. pixelweise bei einem bestimmten Grauwertübergang bzw. Schwellwert auf die Kontur 24 des Behältnisses 3 geschlossen wird. Anschließend werden nur solche Bildpunkte ausgewertet, die innerhalb der ermittelten Kontur 24 des Behältnisses 3 liegen. Der Grauwert des jeweiligen Bildpunkts, der innerhalb der Kontur liegt, wird zur Erstellung einer Referenzlinie herangezogen. Der jeweilige Grauwert wird mit demjenigen Grauwert 38 des Referenzobjekts 35 verglichen, dessen zugehörige Dicke bekannt ist. Damit wird dem jeweiligen Bildpunkt eine Dicke zugeordnet. Dies erfolgt für alle innerhalb der Kontur 24 liegenden Bildpunkte, woraus sich für die bekannte Fläche der Kontur 24 eine mittlere Dicke ergibt. Diesem Volumen lässt sich ein Gewicht zuordnen. Hierzu erfasst eine Wiegeeinrichtung 44 das Gewicht des leeren Behältnisses 3. Man spricht hierbei von der Tarajustierung. Die Dämpfungkurve stellt den Zusammenhang her zwischen den jeweiligen Grauwerten des Bildes 15 bzw. Bildpunktes und dem zugehörigen Leergewicht des Behältnisses 3.

In einem zweiten Schritt 102 wird das System auf die im ersten Schritt 101 eingelernten Behältnisse 3, nun allerdings befüllt mit Produkt 2, eingelernt. Wiederum wird eine Referenzlinie von dem Behältnis 3 mit Produkt 2 erstellt wie bereits oben (für ein leeres Behältnis 3 beschrieben), wobei der Bezug zum gravimetrischen Gewicht mit der Wiegeeinrichtung 44 hergestellt wird, die das Gewicht eines mit Produkt 2 befüllten Behältnisses 3 ermittelt. In dieser Weise erfolgt die sog. Bruttojustierung. Wiederum werden die jeweiligen Grauwerte über die ermittelte Referenzlinie dem entsprechenden Gewicht eines befüllten Behältnisses 3 zugeordnet. Diese Einlernschritte 101 und 102 werden vor Beginn der Produktionsphase einer neuen Charge ausgeführt, die sich von der vorhergehenden sowohl in der Art des Behältnisses 3 und/oder des befüllten Produkts 2 unterscheidet. Diese Schritte 101 und 102 können mit mehreren, unterschiedlichen Behältnissen 3 durchgeführt werden und die Ergebnisse geeignet gemittelt bzw. interpoliert werden.

Alternativ könnte vorgesehen sein, dass die Betriebsparameter einer einmalig auf bestimmte Chargenparameter eingestellten Vorrichtung 10 (wie beispielweise oben beschrieben) gespeichert und später für eine gleichartige Charge wieder geladen werden können. Dann könnten sich die beschriebenen Justageschritte 101 und 102 erübrigen.

In einem dritten Schritt 103 erzeugt der Sensor 30 ein Bild 12 von dem im Strahlungsgang 36 befindlichen, zu überprüfenden und mit Produkt 2 befüllten Behältnis 3. Das Gewicht des Produkts 2 soll ermittelt werden mittels Leerbehältnisextrapolation.

In einem vierten Schritt 104 wird in dem in Schritt 103 erfassten Bild 12 zumindest eine Auswerteregion 21 festgelegt. Hierzu kann über eine entsprechende Bilderkennung zuerst die Kontur 24 des durchstrahlten Behältnisses 3 ermittelt werden. Anhand der ermittelten Graustufen, der Lage oder der Geometrie des Behältnisses 3 bzw. in Kenntnis des abgefüllten Produkts 2 wird dann die Auswerteregion 21 so gewählt, dass sich in dieser Auswerteregion 21 sicher kein Produkt 2 befindet. Die Festlegung zumindest der Auswerteregion 21 könnte beispielsweise im Rahmen der Einlernphase vom Bedienpersonal über entsprechende Eingaben manuell vorgenommen werden. Alternativ könnte diese Festlegung auch automatisch basierend auf Grauwert, Kontur 24 des Behältnisses 3 oder Informationen über das zu erwartende Volumen des abgefüllten Produkts 2 erfolgen. Die gewählte Auswerteregion 21 wird einmal festgelegt und für die nachfolgenden Schritte zur Gewichtserfassung beibehalten. Die nachfolgenden Schritte 105 bis 107 werden dann im laufenden Produktionsbetrieb durchgeführt.

In einem fünften Schritt 105 wird die in Schritt 104 festgelegte Auswerteregion 21, nämlich der frei durchleuchtbare Teil des Behältnisses 3, ausgewertet. Die in der Auswerteregion 21 liegenden Bildpunkte werden wie oben beschrieben bezüglich ihrer Grauwerte ausgewertet. Basierend auf dieser Information und der im ersten Schritt 101 bestimmten Referenzlinie für ein leeres Behältnis 3 wird nun das Leergewicht des im dritten Schritt 103 durchleuchteten Behältnisses 3 extrapoliert. So wird das Taragewicht des Behältnisses 3 ermittelt. Dabei wird beispielsweise die Fläche der Auswerteregion 21 ins Verhältnis gesetzt zur Gesamtfläche des Behältnisses 3, um so auf das Gesamtgewicht des leeren Behältnisses 3 zu schließen. Die Gesamtfläche des Behältnisses 3 lässt sich über die erfasste Kontur 24 ermitteln. Die Anzahl der innerhalb der Kontur 24 liegenden Bildpunkte, deren Größe bekannt ist, ist ein Maß für die Gesamtfläche. Aber auch andere Ermittlungsmöglichkeiten wären denkbar. Über das Gewicht des Anteils der Fläche der Auswerteregion 21 bezogen auf die Gesamtfläche des Behältnisses 3 wird extrapoliert auf das Gewicht des kompletten leeren Behältnisses 3, das Taragewicht, das am Ende des Schritts 105 zur Verfügung steht.

In einem sechsten Schritt 106 erfolgt die Auswertung des gesamten Behältnisses 3 inklusive Produkt 2, basierend auf dem im dritten Schritt 103 erfassten Bild 12. Hierzu werden alle innerhalb der Kontur des gesamten Behältnisses 3 liegenden Bildpunkte mit deren Grauwerten ausgewertet. Aus dieser Information und der im zweiten Schritt 102 ermittelten Referenzlinie für ein befülltes Behältnis 3 wird nun das Bruttogewicht des im dritten Schritt 103 durchstrahlten Behältnisses 3 bestimmt. Für die entsprechende Bruttogewichtsbestimmung wird verfahren wie oben allgemein beschrieben. Die Grauwerte von Behältnis 3 und Produkt 2 überlagern sich. Deshalb umfasst die auf den Grauwerten basierende Gewichtsermittlung sowohl das Gewicht des leeren Behältnisses 3 als auch das Gewicht des Produkts 2.

In einem siebten Schritt 107 wird das Nettogewicht des Produkts 2 gemäß der Beziehung ermittelt: Nettogewicht = Bruttogewicht (wie im sechsten Schritt 106 ermittelt) - Taragewicht (wie im fünften Schritt 105 ermittelt). Das Ergebnis ist somit das reine Nettogewicht der Behältnisfüllung, also das Gewicht des Produkts 2.

Durch das beschriebene Vorgehen wird gezielt das Nettogewicht des Produkts 2 ermittelt und nicht mehr lediglich das Bruttogewicht von Behältnis 3 und Produkt 2 wie im Stand der Technik. Dies ist insbesondere bei kleinen Füllmengen von besonderer Bedeutung, da in diesen Fällen das Gewicht des Behältnisses 3 einen großen Einfluss auf die Genauigkeit der Gewichtsermittlung besitzt. So können bei kleinen Füllmengen Fertigungsschwankungen des Taragewichts leerer Behältnisse 3 größer sein als maximal zulässige Füllmengenschwankungen. Dies kann dazu führen, dass Behältnisse 3 mit korrekter Füllmenge des Produkts 2 als nicht korrekt befüllt aussortiert werden, weil das Taragewicht des Behältnisses 3 die Gewichtstoleranz ausgeschöpft hat. Umgekehrt kann dies auch dazu führen, dass ein mit zu viel oder zu wenig befülltem Produkt 2 befülltes Behältnis 3 fälschlicher Weise als in Ordnung erkannt wird, weil sich die tolerierte Behältnisgewichtsschwankung gegenläufig verhält und die Fehldosierung ausgleicht. Diesen nicht akzeptablen Fällen wirkt die beschriebene Nettogewichtsermittlung mittels Leerbehältnisextrapolation entgegen.

Das beschriebene Vorgehen eignet sich insbesondere für pharmazeutische Hartgelatinekapseln, aber auch für Tabletten, Glasbehälter wie Vials, Ampullen, oder ähnliches, ist jedoch hierauf nicht eingeschränkt.

## Patentansprüche

1. Vorrichtung zur Gewichtsermittlung eines mit Produkt (2) befüllten Behältnisses (3), umfassend zumindest eine Röntgenstrahlungsquelle (28), die einen Strahlungsgang (18) erzeugt zur Durchstrahlung des Behältnisses (3), umfassend zumindest einen Sensor (14), der die Strahlung des durchstrahlten Behältnisses (3) in Form eines Bildes (12) erfasst, wobei dass das Bild (12) des durchstrahlten Behältnisses (3) unterteilt ist in zumindest eine Auswerteregion (21), in der sich kein Produkt (2) befindet, wobei eine Auswerteeinrichtung (14) vorgesehen ist, die unter Verwendung der Auswerteregion (21) ein Taragewicht des durchstrahlten Behältnisses (3) ermittelt, **dadurch gekennzeichnet, dass** die Auswerteeinrichtung (14) das Taragewicht des Behältnisses (3) ermittelt, indem die Auswerteeinrichtung (14) ein Maß für eine Fläche der Auswerteregion (21) in das Verhältnis setzt zu einem Maß für eine Fläche einer Kontur (24) des gesamten durchstrahlten Behältnisses (3).

2. Vorrichtung nach Anspruch 1, **dadurch gekennzeichnet, dass** die Auswerteeinrichtung (14) unter Verwendung des Bildes (12) ein Bruttogewicht des durchstrahlten Behältnisses (3) ermittelt, und anschließend ein Nettogewicht des in dem Behältnis (3) befindlichen Produkts (2) ermittelt unter Verwendung des Bruttogewichts und des Taragewichts.

3. Vorrichtung nach einem der vorhergehenden Ansprüche, **dadurch gekennzeichnet, dass** die Auswerteeinrichtung (14) innerhalb der Auswerteregion (21) und/oder der Kontur liegende Grauwerte und/oder Bildpunkte des Sensors (30) auswertet zur Ermittlung des Gewichts.

4. Vorrichtung nach einem der vorhergehenden Ansprüche, **dadurch gekennzeichnet, dass** die Auswerteeinrichtung (14) als Maß für eine Fläche der Auswerteregion (21) bzw. der Kontur (24) solche Bildpunkte des Sensors (30) verwendet, die innerhalb der Auswerteregion (21) bzw. innerhalb der Kontur (24) liegen.

5. Vorrichtung nach einem der vorhergehenden Ansprüche, **dadurch gekennzeichnet, dass** die Röntgenstrahlungsquelle (28) zum Einlernen ein leeres Behältnis (3) und/oder ein mit Produkt (2) befülltes Behältnis (3) durchstrahlt und dass die Auswerteeinrichtung (14) beim Einlernen eine Referenzlinie erstellt, die unterschiedlichen Grauwerten ein entsprechendes Gewicht zuordnet.

6. Vorrichtung nach einem der vorhergehenden Ansprüche, **dadurch gekennzeichnet, dass** eine Wiegeeinrichtung (44) vorgesehen ist, die das Gewicht des Behältnisses (3) erfasst und die Auswerteeinrichtung (14) die Referenzlinie unter Berücksichtigung des erfassten Gewichts ermittelt.

7. Vorrichtung nach einem der vorhergehenden Ansprüche, **dadurch gekennzeichnet, dass** zumindest ein Referenzobjekt (35) vorgesehen ist, das im Strahlungsgang (18) der Röntgenstrahlungsquelle (28) angeordnet ist, und das unterschiedliche Dicken aufweist.

8. Vorrichtung nach einem der vorhergehenden Ansprüche, **dadurch gekennzeichnet, dass** ein Aufnahmemittel, insbesondere ein Kapselhalter (42), vorgesehen ist zur Aufnahme zumindest eines Behältnisses (3), dass das Behältnis (3) über ein Fördermittel (40) zu einer Bearbeitungsstation (43) und/oder der Röntgenstrahlungsquelle (28) zugeführt ist zur Erzeugung des Bildes (12).

9. Verfahren zur Gewichtsermittlung insbesondere eines mit Produkt (2) befüllten Behältnisses (3), wobei zumindest eine Röntgenstrahlungsquelle (28) einen Strahlungsgang (18) erzeugt zur Durchstrahlung des Behältnisses (3), wobei zumindest einen Sensor (14) die Strahlung des durchstrahlten Behältnisses (3) in Form eines Bildes (12) erfasst wobei das Bild (12) des durchstrahlten Behältnisses (3) unterteilt wird in zumindest eine Auswerteregion (21), in der sich kein Produkt (2) befindet, wobei unter Verwendung der Auswerteregion (21) ein Taragewicht des durchstrahlten Behältnisses (3) ermittelt wird, **dadurch gekennzeichnet, dass** das Taragewicht des Behältnisses (3) ermittelt wird, indem ein Maß für eine Fläche der Auswerteregion (21) in das Verhältnis gesetzt wird zu einem Maß für eine Fläche einer Kontur (24) des gesamten durchstrahlten Behältnisses (3).

10. Verfahren nach dem vorhergehenden Verfahrensanspruch, **dadurch gekennzeichnet, dass** unter Verwendung des Bildes (12) ein Bruttogewicht des durchstrahlten Behältnisses (3) ermittelt wird, und anschließend ein Nettogewicht des in dem Behältnis (3) befindlichen Produkts (2) ermittelt wird unter Verwendung des Bruttogewichts und des Taragewichts.

11. Verfahren nach einem der vorhergehenden Verfahrensansprüche, **dadurch gekennzeichnet, dass** innerhalb der Auswerteregion (21) und/oder Kontur (24) liegende Grauwerte und/oder Bildpunkte des Sensors (30) ausgewertet werden zur Ermittlung des Gewichts und/oder zur Ermittlung eines Maßes für eine Fläche der Auswerteregion (21) und/oder Kontur (24).

12. Verfahren nach einem der vorhergehenden Verfahrensansprüche, **dadurch gekennzeichnet, dass** zum Einlernen ein leeres Behältnis (3) und/oder ein mit Produkt (2) befülltes Behältnis (3) durchstrahlt und dass beim Einlernen eine Referenzlinie erstellt wird, die unterschiedlichen Grauwerten ein entsprechendes Gewicht zuordnet.

13. Verfahren nach einem der vorhergehenden Verfahrensansprüche, **dadurch gekennzeichnet, dass** zumindest ein Referenzobjekt (35) durchstrahlt wird und hierbei entstehende Grauwerte (38) entsprechende Dicken des Referenzobjekts (35) zugeordnet sind, wobei zur Tara- und/oder Bruttogewichtsermittlung die Grauwerte des Sensors (30) der dem jeweiligen Grauwert (38) des Referenzobjekts (35) entsprechenden Dicke des Referenzobjekts (35) zugeordnet wird.

## Claims

1. Apparatus for weight determination of a container (3) filled with product (2), comprising at least one X-ray radiation source (28) which generates a radiation path (18) for radiating the container (3), comprising at least one sensor (14) which detects the radiation of the irradiated container (3) in the form of an image (12), wherein the image (12) of the irradiated container (3) is subdivided into at least one evaluation region (21) in which no product (2) is located, wherein an evaluation device (14) is provided, which determines a tare weight of the irradiated container (3) using the evaluation region (21), **characterised in that** the evaluation device (14) determines the tare weight of the container (3) **in that** the evaluation device (14) relates a measure of an area of the evaluation region (21) to a measure of an area of a contour (24) of the entire irradiated container (3) .

2. Apparatus according to claim 1, **characterized in that** the evaluation device (14) determines a gross weight of the irradiated container (3) using the image (12), and then determines a net weight of the product (2) located in the container (3) using the gross weight and the tare weight.

3. Apparatus according to any of the preceding claims, **characterized in that** the evaluation device (14) evaluates grey values and/or pixels of the sensor (30), which lie within the evaluation region (21) and/or the contour to determine the weight.

4. Apparatus according to any of the preceding claims, **characterized in that** the evaluation device (14) uses as a measure for an area of the evaluation region (21) or of the contour (24) those pixels of the sensor (30), which lie within the evaluation region (21) or within the contour (24).

5. Apparatus according to any of the preceding claims, **characterized in that** the X-ray radiation source (28) for teaching-in radiates an empty container (3) and/or a container (3) filled with product (2) and that the evaluation device (14) generates a reference line during the teaching-in, which assigns a corresponding weight to different grey values.

6. Apparatus according to any of the preceding claims, **characterized in that** a weighing device (44) is provided, which detects the weight of the container (3) and the evaluation device (14) determines the reference line taking into account the detected weight.

7. Apparatus according to any of the preceding claims, **characterized in that** at least one reference object (35) is provided, which is arranged in the radiation path (18) of the X-ray radiation source (28) and which has different thicknesses.

8. Apparatus according to any of the preceding claims, **characterized in that** a receiving means, in particular a capsule holder (42), is provided for receiving at least one container (3), **in that** the container (3) is conveyed via a conveying means (40) to a processing station (43) and/or the X-ray source (28) for producing the image (12) .

9. Method for weight determination in particular of a container (3) filled with product (2), wherein at least one X-ray radiation source (28) generates a radiation path (18) for radiating the container (3), wherein at least one sensor (14) detects the radiation of the irradiated container (3) in the form of an image (12), wherein the image (12) of the irradiated container (3) is subdivided into at least one evaluation region (21) in which no product (2) is located, wherein a tare weight of the irradiated container (3) is determined using the evaluation region (21), **characterized in that** the tare weight of the container (3) is determined **in that** a measure of an area of the evaluation region (21) is related to a measure of an area of a contour (24) of the entire irradiated container (3).

10. Method according to the preceding claim, **characterized in that** a gross weight of the irradiated container (3) is determined using the image (12) and then a net weight of product (2) located in the container (3) is determined using the gross weight and the tare weight.

11. Method according to any of the preceding method claims, **characterized in that** grey values and/or pixels of the sensor (30), which lie within the evaluation region (21) and/or contour (24) are evaluated to determine a weight and/or a measure of an area of the evaluation region (21) and/or contour (24).

12. Method according to any of the preceding method claims, **characterized in that** for teaching-in an empty container (3) and/or a container (3) filled with product (2) is radiated and that during the teaching-in a reference line is generated which assigns a corresponding weight to different grey values.

13. Method according to any of the preceding method claims, **characterized in that** at least one reference object (35) is radiated and grey values (38) resulting therefrom are assigned to corresponding thicknesses of the reference object (35), wherein the grey values of the sensor (30) being assigned to the thickness of the reference object (35) corresponding to the respective grey value (38) of the reference object (35) for tare and/or gross weight determination.

## Revendications

1. Dispositif de détermination de poids d'un récipient (3) rempli de produit (2), comprenant au moins une source de rayonnement X (28) qui génère un trajet de rayonnement (18) destiné à passer à travers le récipient (3), comprenant au moins un capteur (14) qui enregistre le rayonnement du récipient (3) radiographié sous la forme d'une image (12), dans lequel l'image (12) du récipient (3) radiographié est subdivisée en au moins une région d'évaluation (21) dans laquelle il n'y a pas de produit (2), dans lequel un dispositif d'évaluation (14) est prévu qui détermine une tare du récipient (3) radiographié en utilisant ladite région d'évaluation (21), **caractérisé en ce que** le dispositif d'évaluation (14) détermine la tare du récipient (3) par le fait que le dispositif d'évaluation (14) met en rapport une mesure pour une surface de ladite région d'évaluation (21) avec une mesure pour une surface d'un contour (24) de l'ensemble du récipient (3) radiographié.

2. Dispositif selon la revendication 1, **caractérisé par le fait que** le dispositif d'évaluation (14) détermine un poids brut du récipient (3) radiographié en utilisant l'image (12), et puis détermine un poids net du produit (2) contenu dans le récipient (3) en utilisant le poids brut et la tare.

3. Dispositif selon l'une quelconque des revendications précédentes, **caractérisé par le fait que** le dispositif d'évaluation (14) évalue des valeurs de gris et/ou des pixels du capteur (30) qui sont situés à l'intérieur de la zone d'évaluation (21) et/ou du contour afin de déterminer le poids.

4. Dispositif selon l'une quelconque des revendications précédentes, **caractérisé par le fait que** le dispositif d'évaluation (14) utilise en tant que mesure pour une surface de la région d'évaluation (21) ou bien du contour (24) de tels pixels du capteur (30) qui se trouvent à l'intérieur de la région d'évaluation (21) ou bien à l'intérieur du contour (24).

5. Dispositif selon l'une quelconque des revendications précédentes, **caractérisé par le fait que**, pour l'apprentissage, la source de rayonnement X (28) fait passer les rayons à travers un récipient (3) vide et/ou un récipient (3) rempli de produit (2), et que, pendant l'apprentissage, ledit dispositif d'évaluation (14) établit une ligne de référence qui associe un poids correspondant à différentes valeurs de gris.

6. Dispositif selon l'une quelconque des revendications précédentes, **caractérisé par le fait qu'**un dispositif de pesée (44) est prévu qui acquiert le poids du récipient (3) et que le dispositif d'évaluation (14) détermine la ligne de référence en tenant compte du poids acquis.

7. Dispositif selon l'une quelconque des revendications précédentes, **caractérisé par le fait qu'**au moins un objet de référence (35) est prévu qui est disposé dans le trajet de rayonnement (18) de la source de rayonnement X (28) et qui présente des épaisseurs différentes.

8. Dispositif selon l'une quelconque des revendications précédentes, **caractérisé par le fait qu'**un moyen de réception, en particulier un porte-capsule (42), est prévu destiné à recevoir au moins un récipient (3), que le récipient (3) est amené par l'intermédiaire d'un moyen de transport (40) à un poste de traitement (43) et/ou à la source de rayonnement X (28) pour générer l'image (12).

9. Procédé de détermination de poids en particulier d'un récipient (3) rempli de produit (2), dans lequel au moins une source de rayonnement X (28) génère un trajet de rayonnement (18) destiné à passer à travers le récipient (3), dans lequel au moins un capteur (14) enregistre le rayonnement du récipient (3) radiographié sous la forme d'une image (12), dans lequel l'image (12) du récipient (3) radiographié est subdivisée en au moins une région d'évaluation (21) dans laquelle il n'y a pas de produit (2), dans lequel une tare du récipient (3) radiographié est déterminée en utilisant ladite région d'évaluation (21), **caractérisé en ce que** la tare du récipient (3) est déterminée par le fait qu'une mesure pour une surface de ladite région d'évaluation (21) est mis en rapport avec une mesure pour une surface d'un contour (24) de l'ensemble du récipient (3) radiographié.

10. Procédé selon la revendication de procédé précédente, **caractérisé par le fait qu'**un poids brut du récipient (3) radiographié est déterminé en utilisant l'image (12), et que ensuite un poids net du produit (2) contenu dans le récipient (3) est déterminé en utilisant le poids brut et la tare.

11. Procédé selon l'une quelconque des revendications de procédé précédentes, **caractérisé par le fait que** des valeurs de gris et/ou des pixels du capteur (30) qui sont situés à l'intérieur de la zone d'évaluation (21) et/ou du contour (24) sont évalués pour déterminer le poids et/ou pour déterminer une mesure pour une surface de la zone d'évaluation (21) et/ou du contour (24).

12. Procédé selon l'une quelconque des revendications de procédé précédentes, **caractérisé par le fait que**, pour l'apprentissage, on fait passer les rayons à travers un récipient (3) vide et/ou un récipient (3) rempli de produit (2), et que, pendant l'apprentissage, une ligne de référence est établie qui associe un poids correspondant à différentes valeurs de gris.

13. Procédé selon l'une quelconque des revendications de procédé précédentes, **caractérisé par le fait qu'**au moins un objet de référence (35) est radiographié et que des valeurs de gris (38) en résultant sont associées à des épaisseurs correspondantes de l'objet de référence (35), dans lequel, pour déterminer la tare et/ou le poids brut, les valeurs de gris du capteur (30) sont associées à l'épaisseur de l'objet de référence (35), qui correspond à la valeur de gris (38) respective de l'objet de référence (35).
